# EUROPÄISCHE PATENTANMELDUNG

(11) **EP 2 301 675 A1**
(43) Veröffentlichungstag der Anmeldung: **30.03.2011**
(21) Anmeldenummer: 10009801.1
(22) Anmeldetag: 17.09.2010
(51) Int. Cl.: B05B 11/02, B05B 11/00

(54) **Spender mit eine von aussen betätigbare Ventileinrichtung und Montageverfahren für einen solchen Spender**

(30) Priorität: 29.09.2009 DE 102009048551
(71) Anmelder: Ing. Erich Pfeiffer GmbH, 78315 Radolfzell (DE)
(72) Erfinder: Grenier-Perth, Jürgen, 78244 Gottmadingen (DE)
(74) Vertreter: Patentanwälte Ruff, Wilhelm, Beier, Dauster & Partner

(57) **Zusammenfassung**

Die Erfindung betrifft primär eine Austragvorrichtung (10) mit einem Außengehäuse (20), mit einer Austragöffnung (66), mit einem Flüssigkeitsspeicher (80), mit einem Auslasskanal (58, 56, 70, 74), durch den der Flüssigkeitsspeicher (80) mit der Austragöffnung (66) verbunden ist, und mit einer gegenüber dem Außengehäuse (20) verlagerbaren Betätigungshandhabe (82). Dabei ist die Betätigungshandhabe (82) mit dem Flüssigkeitsspeicher (80) derart mechanisch gekoppelt, dass eine Verlagerung der Betätigungshandhabe (82) eine Volumenreduktion des Flüssigkeitsspeichers (80) bewirkt.
Eine Ventileinrichtung (54, 72) ist dem Auslasskanal (58, 56, 70, 74) zugeordnet, die in einem Öffnungszustand die Verbindung des Flüssigkeitsspeichers (80) mit der Austragöffnung (66) freigibt und in einem Schließzustand die Verbindung des Flüssigkeitsspeichers (88) mit der Austragöffnung (66) trennt, und dass die Ventileinrichtung (54, 72) eine von außen zugängliche Ventilhandhabe (68) aufweist, mittels derer die Ventileinrichtung (54, 72) von außen aus dem Öffnungszustand in den Schließzustand überführbar ist.

## Beschreibung

### Anwendungsgebiet und Stand der Technik

Die Erfindung betrifft eine gattungsgemäße Austragvorrichtung mit einem Außengehäuse, einer Austragöffnung, einem Flüssigkeitsspeicher, einem Auslasskanal, durch den der Flüssigkeitsspeicher mit der Austragöffnung verbunden ist oder verbunden werden kann, und einer gegenüber dem Außengehäuse verlagerbaren Betätigungshandhabe. Dabei ist bei einer gattungsgemäßen Austragvorrichtung die Betätigungshandhabe mit dem Flüssigkeitsspeicher derart mechanisch gekoppelt, dass eine Verlagerung der Betätigungshandhabe eine Volumenreduktion des Flüssigkeitsspeichers bewirkt. Die Erfindung betrifft weiterhin ein Montageverfahren hierfür.

Eine gattungsgemäße Austragvorrichtung ist beispielsweise aus der DE 44 12 041 A1 bekannt. Die Besonderheit bei gattungsgemäßen Austragvorrichtungen liegt darin, dass keine vom Flüssigkeitsspeicher separate Fördereinrichtung wie beispielsweise eine Förderpumpe vorgesehen ist, die das Medium aus dem Flüssigkeitsspeicher per Unterdruck ansaugt und zur Austragöffnung fördert, sondern dass stattdessen das Volumen des Flüssigkeitsspeichers selbst reduziert wird, um die Flüssigkeit unmittelbar zur Austragöffnung zu drücken.

Da gattungsgemäße Austragvorrichtungen vorzugsweise als Einweg-Austragvorrichtungen zur Ausbringung nur einer Flüssigkeitscharge vorgesehen sind, ist es von besonderer Wichtigkeit, dass die Luftmenge im Flüssigkeitskanal zwischen dem Flüssigkeitsspeicher und der Austragöffnung im Lieferzustand der Austragvorrichtung gering ist, vorzugsweise null ist, um bei einer Betätigung der Betätigungshandhabe durch einen Nutzer unmittelbar einen Medienaustrag zu bewirken.

Bei der gattungsgemäßen DE 44 12 041 ist zu diesem Zweck ein besonders dünner Medienkanal vorgesehen, der durch eine Hohlnadel gebildet wird, die bei der Betätigung der Austragvorrichtung durch einen Stopfen im Flüssigkeitsspeicher hindurch dringt und unter Verlagerung dieses Stopfens das Medium aus dem Flüssigkeitsspeicher zur Austragöffnung fördert.

Diese Bauweise hat sich zwar als zuverlässig erwiesen, sie ist jedoch hinsichtlich der Herstellungskosten nachteilig.

### Aufgabe und Lösung

Aufgabe der Erfindung ist es daher, eine kostengünstige Austragvorrichtung zur Verfügung zu stellen, die auf einfache Weise bereits während der Montage in einen Zustand versetzt werden kann, in dem ein unmittelbarer Austrag von Flüssigkeit möglich ist.

Erfindungsgemäß wird dies dadurch erreicht, dass dem Auslasskanal eine Ventileinrichtung zugeordnet ist, die in einem Öffnungszustand die Verbindung des Flüssigkeitsspeichers mit der Austragöffnung freigibt und die in einem Schließzustand die Verbindung des Flüssigkeitsspeichers mit der Austragöffnung trennt. Dabei ist vorgesehen, dass die Ventileinrichtung eine von außen zugängliche Ventilhandhabe aufweist, mittels derer die Ventileinrichtung von außen aus dem Öffnungszustand in den Schließzustand überführbar ist.

Die Ventilhandhabe wirkt dabei unmittelbar, d.h. ohne mechanische Zwischenschaltung der Flüssigkeit in der Austragvorrichtung, auf die Ventileinrichtung und erlaubt es somit, im Öffnungszustand der Ventileinrichtung den mit Flüssigkeit befüllten Flüssigkeitsspeicher an die anderen Bauteile der Austragvorrichtung zu koppeln und dabei zumindest einen Teil des Auslasskanals bereits mit Flüssigkeit zu befüllen. Erst abschließend wird über die Ventilhandhabe die Ventileinrichtung geschlossen, um dadurch einen Transport- und Auslieferungszustand herzustellen, in dem die Flüssigkeit gegenüber der Umgebung geschützt ist, insbesondere vor Kontamination geschützt ist.

Unter der Zugänglichkeit der Ventilhandhabe von außen wird im Zusammenhang mit dieser Erfindung verstanden, dass im angekoppelten Zustand des Flüssigkeitsspeichers durch eine von außen bewirkte Kraftbeaufschlagung oder durch den Wegfall einer von außen zuvor bewirkten Kraftbeaufschlagung an einer von der Betätigungshandhabe getrennt beweglichen Ventilhandhabe der Schließzustand herbeigeführt werden kann.

Als Flüssigkeitsspeicher wird im Zusammenhang mit der vorliegenden Erfindung ein zur Aufnahme der Flüssigkeit vor einem Austragvorgang vorgesehener Behälter angesehen, der vorzugsweise keinen Einlasskanal aufweist, sondern lediglich eine Austragöffnung, durch die er im an die Austragvorrichtung angekoppelten Zustand mit dem Auslasskanal verbunden ist. Das Innenvolumen dieses Flüssigkeitsspeichers, genauer das die auszubringenden Flüssigkeit beinhaltende Volumen, kann durch Relativverlagerung des Flüssigkeitsspeichers gegenüber einem Kolbenelement verringert werden.

Eine besonders einfache und bevorzugte Ausführungsform sieht vor, dass der Flüssigkeitsspeicher einteilig, d.h. stets ortsfest, mit der Betätigungshandhabe ausgebildet ist und dass ein zum Außengehäuse ortsfester Kolbenabschnitt vorgesehen ist, der bei einer über die Betätigungshandhabe bewirkten Verlagerung des Flüssigkeitsspeichers in den Flüssigkeitsspeicher einrückt. Gattungsgemäße Spender mit diesen Merkmalen sind bekannt. Insbesondere bei einer solchen Gestaltung einer Austragvorrichtung ist die erfindungsgemäß vorgesehene Ventileinrichtung von Vorteil, da sie es gestattet, dass während der Montage ohne Kompression der in der Austragvorrichtung vorhandene Luft eine Verlagerung des Flüssigkeitsspeichers gegenüber dem Kolbenabschnitt möglich ist, nachdem der Kolbenabschnitt bereits in den Flüssigkeitsspeicher eingerückt ist.

Besonders von Vorteil ist eine Gestaltung, bei der die Ventileinrichtung zwei gegeneinander verlagerbare Ventilabschnitte aufweist, die im Schließzustand der Ventileinrichtung aneinander anliegend den Auslasskanal sperren, wobei die Ventilhandhabe vorzugsweise einteilig, insbesondere einstückig, mit einem der Ventilabschnitte ausgebildet.

Diese Bauweise ist besonders zuverlässig, da durch die Verlagerung der Ventilhandhabe unmittelbar auch der damit einteilig verbundene Ventilabschnitt verlagert wird. Zudem ist diese Bauweise auch sehr kostengünstig, da nur ein Bauteil als Ventilhandhabe und Ventilabschnitt benötigt wird, während der andere Ventilabschnitt beispielsweise einstückig mit dem Außengehäuse oder einem in das Außengehäuse eingesetzten Einsatz ausgebildet sein kann.

Besonders bevorzugt ist es, wenn die Ventileinrichtung als Verwirbelungseinrichtung ausgebildet ist, durch die die Flüssigkeit beim Durchqueren der Ventileinrichtung im Öffnungszustand einen Drall erhält, mittels dessen ein konischer Sprühstrahl realisierbar ist. Besonders vorteilhaft ist es, wenn die Ventilabschnitte in einer Verlagerungsrichtung gegeneinander verlagerbar sind, ein erster Ventilabschnitt als Verlagerungsrichtung erstreckter zylindrischer oder konischer Ventilstift und ein zweiter Ventilabschnitt als eine zumindest im Schließzustand den Ventilstift anliegend umgebende und in Verlagerungsrichtung erstreckende zylindrische oder konische Ventilhülse ausgebildet ist und wenn weiterhin die Ventilhülse in Radialrichtung erstreckte Verwirbelungsdurchbrechungen aufweist.

Bei einer solchen Gestaltung werden die Verwirbelungsdurchbrechungen durch die axiale Verlagerung des Ventilstiftes gegenüber der Ventilhülse in Richtung des Öffnungszustands freigegeben, so dass das Medium durch die Verwirbelungsdurchbrechungen, die vorzugsweise eine Erstreckungsrichtungskomponente in Tangential- und eine Erstreckungsrichtungskomponente in Radialrichtung aufweisen, hindurchströmt.

Es wird als besonders vorteilhaft angesehen, wenn die Ventilhülse an jenem Ventilabschnitt vorgesehen ist, der gegenüber dem Außengehäuse bei der Überführung aus dem Öffnungszustand in den Schließzustand verlagert wird.

Die Austragöffnung der Austragvorrichtung kann ortsfest am Außengehäuse vorgesehen sein. Von Vorteil ist es jedoch, wenn die Austragöffnung in einem gegenüber dem Außengehäuse verlagerbaren Ventilabschnitt vorgesehen ist. Hierdurch wird erreicht, dass das Außengehäuse mit lediglich einer Durchbrechung ausgebildet sein kann, die Zugriff auf die innerhalb des Außengehäuses angeordnete Ventileinrichtung über die in der Durchbrechung angeordnete Ventilhandhabe gestattet und die gleichzeitig der Durchquerung der Flüssigkeit während des Austragvorgangs dient.

Die Ventileinrichtung kann monostabil vorgesehen sein, so dass lediglich der Schließzustand der Ventileinrichtung ohne Aufrechterhaltung einer externen Kraftbeaufschlagung bzw. Aufrechterhaltung eines bestimmten Flüssigkeitsdrucks erhalten bleibt. Von Vorteil ist es jedoch, wenn die Ventileinrichtung bistabil ausgebildet ist, d.h. ihren Öffnungszustand und ihren Schließzustand ohne hierfür erforderliche externe Kraft- oder Druckbeaufschlagung aufrecht erhält. Dadurch kann während der Montage vor dem Ankoppeln des Flüssigkeitsspeichers der Öffnungszustand der Ventileinrichtung hergestellt werden, ohne dass dieser auf umständliche Weise während des Ankoppelns des Flüssigkeitsspeichers gehalten werden muss.

Von besonderem Vorteil ist es weiterhin, wenn die Ventileinrichtung dafür ausgebildet ist, zumindest mittelbar durch Kraftbeaufschlagung der Betätigungshandhabe aus dem Schließzustand in den Öffnungszustand überführbar zu sein. Dies bietet den Vorteil, dass der Benutzer die Ventileinrichtung nicht gezielt manuell in den Öffnungszustand versetzen muss, bevor er die Flüssigkeit austrägt. Stattdessen kann er mit einer einzigen Kraftbeaufschlagung der Betätigungshandhabe die Ventileinrichtung in den Öffnungszustand versetzen und die Flüssigkeit austragen. Auch wenn eine unmittelbare mechanische Kopplung der Betätigungshandhabe mit der Ventileinrichtung hierfür eine Gestaltungsmöglichkeit darstellt, wird es als vorteilhaft angesehen, wenn die Ventileinrichtung dafür ausgebildet ist, mittelbar über den Flüssigkeitsdruck der auszutragenden Flüssigkeit geöffnet zu werden. Dieser Flüssigkeitsdruck wird durch die Kraftbeaufschlagung der Betätigungshandhabe und die dadurch bewirkte Volumenreduzierung des Flüssigkeitsspeichers bewirkt. Konstruktiv wird dies durch eine gegenüber der Verlagerungsrichtung der Ventilabschnitte angestellte Druckbeaufschlagungsfläche an dem gegenüber dem Außengehäuse verlagerbaren Ventilabschnitt erzielt, deren Druckbeaufschlagung die Verlagerung bewirkt.

Die genannte Gestaltung ist auch deshalb von Vorteil, da die Ventileinrichtung so ausgebildet sein kann, dass die zur Überführung in den Öffnungszustand erforderliche Betätigungskraft an der Betätigungshandhabe größer ist als die erforderliche Kraft, um die Flüssigkeit durch den Auslasskanal und die Austragöffnung hindurchzudrücken. Dadurch ergibt sich eine Druckpunktwirkung, d.h. dass das Maximum der erforderlichen Betätigungskraft ist unmittelbar zu Beginn des Austragvorgangs gegeben. Die vom Benutzer hierfür aufzubringende Kraft ist so groß, dass gewährleistet ist, dass der Benutzer nach dem Öffnen der Ventileinrichtung durch die Betätigungskraft den Austragvorgang nicht unterbricht. Die Druckpunktwirkung kann durch zusätzliche im Schließzustand kraftschlüssig wirkende Haltemittel, wie beispielsweise elastisch federnde Rastnasen, erhöht werden.

Insbesondere im Zusammenhang mit einer solchen Ventileinrichtung, die zumindest mittelbar über die Betätigungshandhabe in ihren Öffnungszustand überführt werden kann, ist es von Vorteil, wenn die Ventilhandhabe derart ausgebildet und/oder angeordnet ist, dass von außen und mittels der Ventilhandhabe keine Überführung der Ventileinrichtung aus dem Schließzustand in den Öffnungszustand möglich ist. Somit ist die genannte Überführung in den Öffnungszustand bestimmungsgemäß ausschließlich über die Betätigungshandhabe möglich. Die Ventilhandhabe ist im Schließzustand der Austragvorrichtung durch einen Benutzer nicht verlagerbar, so dass der Benutzer nicht fälschlicherweise den Öffnungszustand vor Beginn des Austragvorgangs herstellen kann. Damit ist die Aufrechterhaltung des Schließzustandes und damit der kontaminationshindernden Wirkung bis unmittelbar vor dem Austragvorgang gewährleistet.

Konstruktiv lässt sich die Unterbindung einer manuellen Öffnung der Ventileinrichtung auf verschiedene Arten realisieren.

So ist es insbesondere möglich, dass die Ventilhandhabe in einer Durchbrechung des Außengehäuses angeordnet ist und im Schließzustand der Ventileinrichtung gegenüber einer Außenfläche des Außengehäuses zumindest partiell zurückgesetzt ist. Im geschlossenen Zustand der Ventilhandhabe ist somit ausschließlich oder fast ausschließlich eine Stirnfläche der Ventilhandhabe von außen zugänglich, welche aber mangels eines geeigneten Angreifpunktes aus der Durchbrechung nicht herausgezogen werden kann.

Es kann allerdings auch ausreichen, die Ventilhandhabe derart auszubilden, dass diese zwar aus der Durchbrechung nach außen hindurchragt, jedoch keine oder zumindest keine ausreichend großen Griffflächen aufweist, die zur Krafteinleitung in Öffnungsrichtung geeignet wären. So kann beispielsweise eine Ventilhandhabe mit zylindrischer Außenform, die zum Öffnen der Ventileinrichtung in Richtung ihrer von außen zugänglichen Stirnfläche gedrückt werden muss, kaum missbräuchlich gehandhabt werden, wenn sie um weniger als 5 mm, insbesondere um weniger als 3 mm, über eine Außenfläche des umgebenden Gehäuses hinausragt. Die zugängliche Mantelfläche reicht nicht aus, um die Ventilhandhandhabe aus der Durchbrechung herauszuziehen. Vorzugsweise ist die Ventilhandhabe zur Erschwerung einer missbräuchlichen Verlagerung in Öffnungsrichtung zumindest im Bereich ihrer von außen zugänglichen Fläche hinterschneidungsfrei bzgl. ihrer Bewegungsrichtung ausgebildet.

Auch eine besonders kleine Ventilhandhabe, beispielsweise mit einer Betätigungsfläche von weniger als 50 mm², ist geeignet, um derart angebracht zu werden, dass sie lediglich in Schließrichtung, nicht aber in Öffnungsrichtung manuell gehandhabt werden kann.

Die Erfindung betrifft weiterhin auch ein Montageverfahren für eine Austragvorrichtung oben genannter Art. Bei diesem Montageverfahren wird zunächst in beliebiger Reihenfolge die Ventileinrichtung in ihren Öffnungszustand versetzt und der zumindest teilweise mit Flüssigkeit gefüllte Flüssigkeitsspeicher an den Auslasskanal angekoppelt, so dass der Flüssigkeitsspeicher mit einer Außenumgebung nur noch über die Austragöffnung verbunden ist. Anschließend wird das Volumen des Flüssigkeitsspeichers verringert. Dies dient insbesondere der Einbringung von Flüssigkeit aus dem Flüssigkeitsspeicher in einen Teil oder den gesamten Auslasskanal. Abschließend wird die Ventileinrichtung über die Ventilhandhabe in ihren Schließzustand versetzt.

### Kurzbeschreibung der Zeichnungen

Weitere Aspekte und Vorteile der Erfindung ergeben sich außer aus den Ansprüchen auch aus der nachfolgenden Beschreibung eines bevorzugten Ausführungsbeispiels der Erfindung, welches in den Figuren dargestellt ist. Dabei zeigen:
- Fig. 1: eine erfindungsgemäße Austragvorrichtung in einer ge- schnittenen Darstellung und
- Fig. 2 bis 5: einen Montage- und einen Austragvorgang der Austrag- vorrichtung der Fig. 1.

### Detaillierte Beschreibung der Ausführungsbeispiele

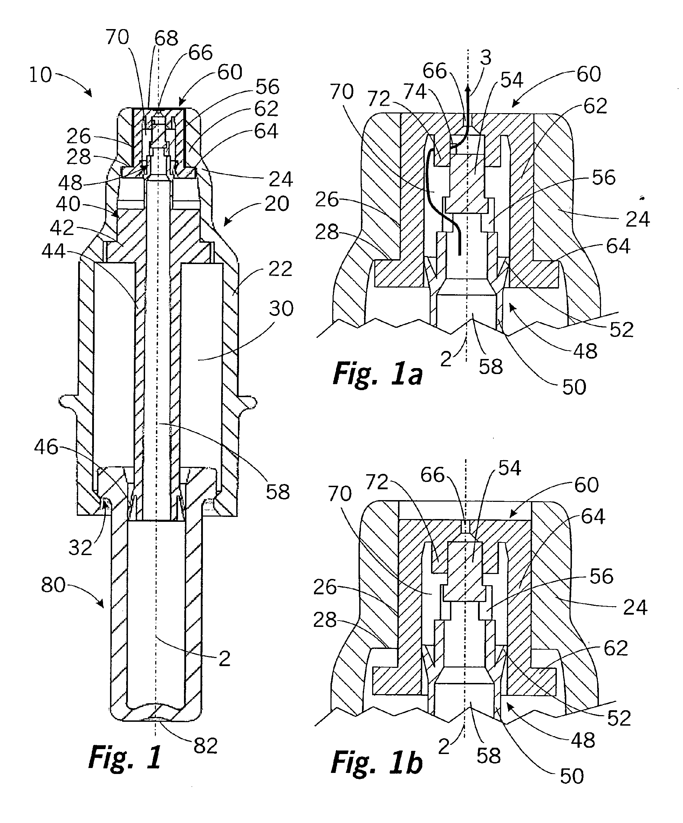

Fig. 1 zeigt eine erfindungsgemäße Austragvorrichtung 10.

Diese Austragvorrichtung 10 besteht aus insgesamt vier separaten Kunststoffteilen. Diese sind ein Außengehäuse 20, ein Einsatz 40, ein Ventilkörper 60 und ein Flüssigkeitsspeicher 80.

Das Außengehäuse 20 weist eine etwa hülsenförmige Grundform auf, deren unterer Teilabschnitt durch einen weitgehend zylindrischen und einen Aufnahmeraum 30 für den Flüssigkeitsspeicher 80 umgebenden Aufnahmeabschnitt 22 gebildet wird und deren oberer Teilabschnitt durch einen sich nach oben verjüngenden Applikatorabschnitt 24 gebildet wird.

In das Außengehäuse 20 ist der Einsatz 40 fest eingefügt. Obwohl bei der vorliegenden Gestaltung das Außengehäuse 20 und der Einsatz 40 als separate Bauteile vorgesehen sind, ist daher grundsätzlich auch eine einstückige Gestaltung denkbar.

Der Einsatz 40 verfügt über einen Kopplungsabschnitt 42, der der kraftschlüssigen Festlegung innerhalb des Außengehäuses 20 dient. An der unteren Seite dieses Kopplungsabschnitts 40 schließt sich ein Rohrabschnitt 44 an, der sich wiederum am unteren Ende zu einem Kolbenabschnitt 46 aufweitet. An der oberen Seite des Kopplungsabschnitts 42 schließt sich ein Funktionsabschnitt 48 an, der in den Fig. 1 a und 1 b in Detail dargestellt ist. Der untere Teil dieses Funktionsabschnitts 48 wird durch einen Rohrabschnitt 50 gebildet, an dessen Außenseite eine umlaufende Dichtlippe 52 vorgesehen ist. Dieser Rohrabschnitt 50 verjüngt sich nach oben hin und endet in einem zylindrischen Ventilstift 54. In einem Übergangsbereich vom Rohrabschnitt 50 zum Ventilstift 54 sind radiale Öffnungen 56 vorgesehen. Der gesamte Einsatz 40 ist von seinem unteren Ende bis zum Rohrabschnitt 50 durch einen Auslasskanal 58 durchdrungen, der am oberen Ende durch diese Öffnungen 56 und am unteren Ende im Bereich des Kolbenabschnitts 46 offen gestaltet ist. Der Ventilkörper 60 verfügt über einen zylindrischen Hauptabschnitt 62 und weitet sich an seinem unteren Ende durch einen umlaufenden Anschlagssteg 64 auf. Der Hauptabschnitt 62 ist in eine axial erstreckte Durchbrechung 26 des Außengehäuses 20 eingesetzt, wobei die Durchbrechung 26 und der Hauptabschnitt 62 hinsichtlich ihres Durchmesser so aufeinander angepasst sind, dass sie eine leichte Presspassung bilden. Der Ventilkörper 60 ist als nach unten offener Hohlkörper ausgebildet, dessen Innenbereich eine Druckkammer 70 bildet, die nach unten durch die Dichtlippe 52 des in die Druckkammer 70 hineinragenden Funktionsabschnitts 48 des Einsatzes 40 begrenzt wird. An der Innenseite des Ventilkörpers 60 ist ein den Zugang zu einer stirnseitigen Austragöffnung 66 umgebender leicht konischer Steg 72 angeformt, der von Verwirbelungsdurchbrechungen 74 unterbrochen ist.

Der Ventilkörper 60 ist gegenüber dem Verbund bestehend aus dem Außengehäuse 20 und dem Einsatz 40 in Richtung der Hauptachse 2 verlagerbar. In der unteren Endlage des Ventilkörpers 60, dargestellt in Fig. 1b, versperren der Ventilstift 54 und der umlaufende Steg 72 gemeinsam den Flüssigkeitspfad zwischen der Druckkammer 70 und der Austragöffnung 66. Die obere Endlage des Ventilkörpers 60, dargestellt in Fig. 1a, wird durch ein Anliegen des Anschlagssteges 64 an einer Stufe 28 an der Innenseite des Außengehäuses 20 definiert. In dieser oberen Endlage sind die Verwirbelungsdurchbrechung 74 durch den Ventilstift 54 freigegeben, so dass der Flüssigkeitspfad 3 zwischen der Druckkammer 70 und der Austragöffnung 66 offen ist.

Am gegenüberliegenden Ende der Austragvorrichtung 10 ist der Flüssigkeitsspeicher 80 vorgesehen. Dieser Flüssigkeitsspeicher 80 ist als einseitig offener und im Wesentlichen zylindrischer Behälter ausgebildet. Er ragt in der montierten Stellung der Fig. 1 bis in den Aufnahmeraum 30 hinein und ist durch eine Verschnappung 32 gegen Herausrutschen gesichert. Der zum Außengehäuse 20 ortsfeste Rohrabschnitt 44 ragt von oben in den Flüssigkeitsspeicher 80 hinein. Der Innendurchmesser des Flüssigkeitsspeichers 80 ist an den Außendurchmesser des Kolbenabschnitts 46 derart angepasst, dass in diesem Zustand der einzige Flüssigkeitspfad aus dem Flüssigkeitsspeicher 80 hinaus durch den Auslasskanal 58 gegeben ist.

Die grundsätzliche Funktionsweise der Austragvorrichtung 10 ist die Folgende: Die Austragvorrichtung 10 wird zum Austragen von Medium aus dem Flüssigkeitsspeicher 80 so angesetzt, dass der Applikatorabschnitt 24 des Außengehäuses 20 bis in ein Nasenloch eines Patienten hineinreicht. Anschließend wird der Flüssigkeitsspeicher 80 durch eine nach oben gerichtete Kraftbeaufschlagung der an der Unterseite des Flüssigkeitsspeichers 80 vorgesehenen Betätigungshandhabe 82 nach oben verlagert, wobei der Kolbenabschnitt 46 des Einsatzes 40 in den Flüssigkeitsspeicher 80 einrückt und dabei das Volumen des Flüssigkeitsspeichers verringert. Dies bewirkt eine Verdrängung der Flüssigkeit aus dem Flüssigkeitsspeicher 80 in den Auslasskanal 58 und durch die Öffnungen 56 und die Druckkammer 70 hindurch bis zur Austragöffnung 66. Dabei wird die Flüssigkeit beim Durchströmen der Verwirbelungsdurchbrechungen 74 tangential verwirbelt, so dass sie in Form eines konischen Sprühstrahls aus der Austragöffnung 66 austritt.

Die dargestellte Austragvorrichtung 10 wird vorzugsweise zum Austragen in einem einzigen Zuge genutzt. Der Flüssigkeitsspeicher 80 kann gegenüber dem Außengehäuse 20 jedoch auch in mehreren Teilschritten verlagert werden, um mehrere Austragvorgänge zu bewirken. Dies kann bei einer nicht dargestellten Ausführungsform dadurch unterstützt werden, dass zwischen dem Außengehäuse 20 oder dem Einsatz 40 einerseits und dem Flüssigkeitsspeicher 80 andererseits überwindbare Sperrmittel vorgesehen sind, die einen vorherigen Austragvorgang mechanisch beenden und erst nach einer erneuten Kraftbeaufschlagung der Betätigungshandhabe 82 im Rahmen eines weiteren Austragvorgangs einen weiteren Anteil der Flüssigkeit austragen. Andere nicht dargestellte Ausführungsformen umfassen eine vom Flüssigkeitsspeicher getrennte Betätigungshandhabe, die dafür ausgebildet ist, bei aufeinanderfolgenden Betätigungen den Flüssigkeitsspeicher schrittweise immer weiter in Richtung der Austragöffnung zu verlagern und dadurch mit jedem Schritt einen Austragvorgang bewirken.

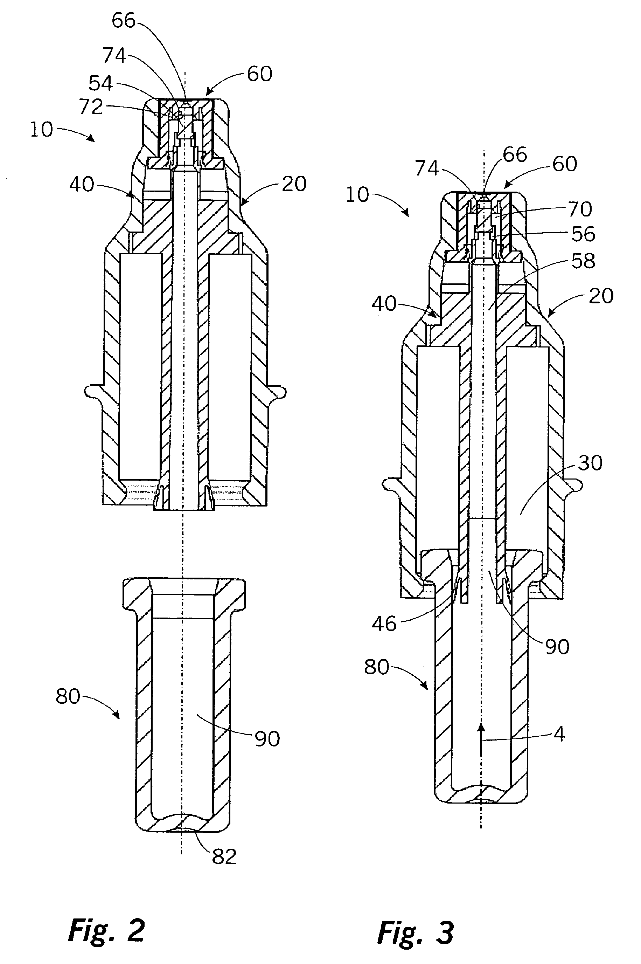

Die Bedeutung des vom Außengehäuse 20 getrennten Ventilkörpers 60 wird nachfolgend anhand der Fig. 2 bis 5 erläutert, die den Montagevorgang und die Nutzung der Austragvorrichtung 10 verdeutlichen.

Fig. 2 zeigt einen Ausgangszustand, in dem der Ventilkörper 60 bereits von unten in das Außengehäuse 20 eingeschoben wurde und in dem der Einsatzes 40 bereits im Außengehäuse 20 fixiert wurde. Der Ventilkörper 60 ist in diesem Zustand der Fig. 2 in seiner der Fig. 1a entsprechenden oberen Endlage, in der die durch den umlaufenden Steg 72 und den Ventilstift 54 gebildete Ventileinrichtung geöffnet ist und die Druckkammer 70 dementsprechend über die Verwirbelungsdurchbrechungen 74 in freier Verbindung mit der Austragöffnung 66 steht.

In diesem Zustand wird der mit einem Medium 90 befüllte Flüssigkeitsspeicher 80 in Richtung des Pfeils 4 von unten in das Außengehäuse 20 eingeschoben, wie die Fig. 3 verdeutlicht. Das Medium 90 wird dabei bereits während der Montage durch den Kolbenabschnitt 46 des Einsatzes 40 zum Teil aus dem Flüssigkeitsspeicher verdrängt und in den Auslasskanal 58 hineingedrückt. Zu einer Erhöhung des Luftdrucks der in der Druckkammer 70 und im Auslasskanal 58 befindlichen Luft kommt es dabei nicht, da die überschüssige Luft aufgrund der geöffneten Ventileinrichtung 54, 72 austreten kann.

Beim dargestellten Montagevorgang ist die montierte Grundstellung des Flüssigkeitsspeichers 80 gegenüber dem Außengehäuse 20 so geartet, dass im Auslasskanal 58 sowie in der Druckkammer 70 noch Luft verbleibt. Bei einer nicht dargestellten Variante des Montagevorgangs wird der Flüssigkeitsspeicher 80 bereits weiter in den Aufnahmeraum 30 des Außengehäuses 20 eingeschoben, so dass die Luft aus dem Auslasskanal 58 und der Druckkammer 70 vollständig aus der Austragvorrichtung herausgedrückt wird.

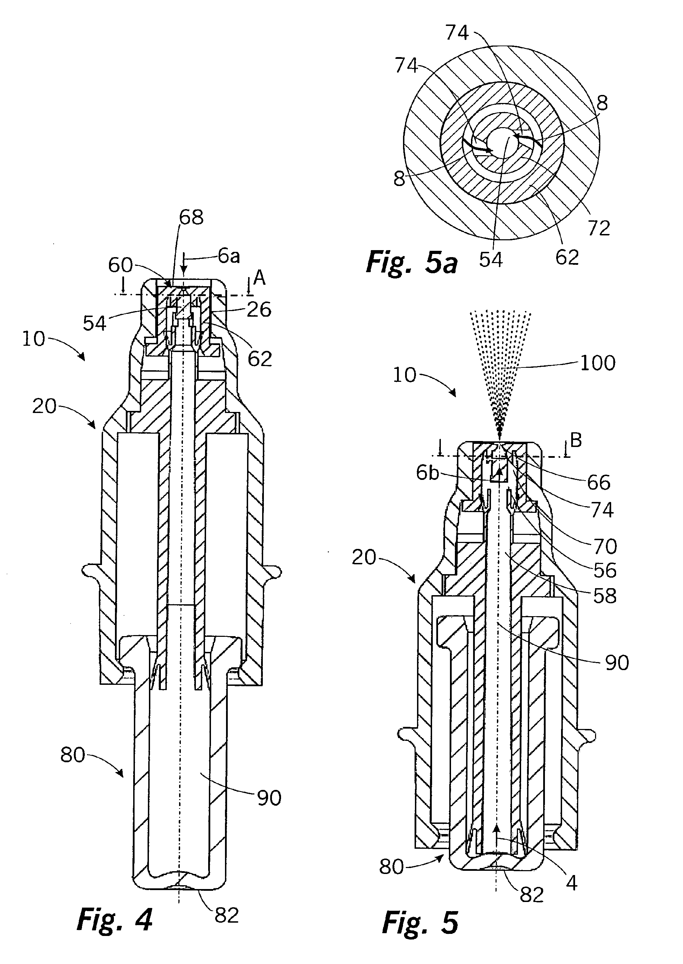

Der Montagevorgang wird dadurch beendet, dass der Ventilkörper 60 in der in Fig. 4 dargestellten Weise tiefer in die Durchbrechung 26 des Außengehäuses 20 hineingedrückt wird. Dies erfolgt durch eine mechanische Kraftbeaufschlagung einer Außenfläche 68 des Ventilkörpers 60 in Richtung des Pfeils 6a. Die Verlagerung erfolgt dabei gegen die Reibungskräfte zwischen der Außenseite des Hauptabschnitts 62 des Ventilkörpers 60 und der Innenseite der Durchbrechung 26, so dass aufgrund dieser Reibungskräfte der in Fig. 4 erreichte verlagerte Zustand des Ventilkörpers 60 kraftschlüssig gesichert ist. Durch die Verlagerung des Ventilkörpers 60 rückt der Ventilstift 54 tiefer in den vom umgebenden Ventilsteg 72 begrenzten Raum ein und verschließt dabei insbesondere die Verwirbelungsdurchbrechungen 74. Die Ventileinrichtung 54, 72 wird dadurch geschlossen, so dass die Flüssigkeit im Flüssigkeitsspeicher 80 und im Auslasskanal 58 gegenüber einer Umgebung steril getrennt ist. In diesem Zustand der Fig. 4 erfolgt die Auslieferung an den Patienten.

Der Patient nutzt die Austragvorrichtung 10 in der eingangs beschriebenen Art und Weise, indem er einen Flüssigkeitsaustrag durch eine Kraftbeaufschlagung der Betätigungshandhabungsfläche 82 des Flüssigkeitsspeichers 80 bewirkt. Eine vorherige unmittelbare manuelle Öffnung der Ventileinrichtung 54, 72 ist aufgrund der gegenüber dem oberen Rand des Außengehäuses zurückgesetzten Lage des Ventilkörpers 60 nicht möglich. Sie ist und auch nicht erforderlich, da bei der bestimmungsgemäßen Betätigung durch die Kraftbeaufschlagung der Betätigungshandhabungsfläche 82 und die damit verbundene Verlagerung des Medienspeichers 80 der Druck innerhalb des Auslasskanals 58 und der Druckkammer 70 ansteigt, bis dieser Druck ausreicht, um die Reibungskräfte zwischen der Außenseite des Hauptabschnitts 62 des Ventilkörpers 60 und der Innenseite der Durchbrechung 26 zu überwinden. Sobald dies der Fall ist, wird der Ventilkörper 60 in Richtung des Pfeils 6b nach oben verlagert, um die Position entsprechend der Fig. 5 und 1 a einzunehmen und dadurch die Ventileinrichtung 54, 72 zu öffnen. Da der hierfür erforderliche Druck erst beim Anliegen einer Mindestbetätigungskraft an der Betätigungshandhabe 82 gegeben ist, die im Wesentlichen durch die Formgebung des Ventilkörpers 60 und der Durchbrechung 26 bestimmt ist, wird der haptische Eindruck eines Druckpunktes erzeugt. Die zur Verlagerung des Ventilkörpers 60 gegenüber der Durchbrechung 26 erforderliche Kraft ist höher als die im dann geöffneten Zustand der Ventileinrichtung 54, 72 erforderliche Kraft zur Ausbringung der Flüssigkeit 90, so dass gewährleistet ist, dass die durch den Patienten zum Öffnen der Ventileinrichtung 54, 72 aufgebrachte Kraft nach Öffnen der Ventileinrichtung einen homogenen und unterbrechungsfreien Austrag des Mediums in einem Zug gewährleistet.

Fig. 5a verdeutlicht die Gestaltung der Verwirbelungsdurchbrechungen 74 im umlaufenden Ventilsteg 72. Nach Öffnen der Ventileinrichtung 54, 72 wird die Flüssigkeit durch diese Durchbrechungen in Richtung der Pfeile 8 hindurchgefördert. Hierbei erhält die Flüssigkeit einen Drall, der in der in Fig. 5 verdeutlichten Art und Weise zu einem konischen Sprühstrahl 100 führt.

## Patentansprüche

1. Austragvorrichtung (10) mit
- einem Außengehäuse (20),
- einer Austragöffnung (66),
- einem Flüssigkeitsspeicher (80),
- einem Auslasskanal (58, 56, 70, 74), durch den der Flüssigkeitsspeicher (80) mit der Austragöffnung (66) verbunden ist, und
- einer gegenüber dem Außengehäuse (20) verlagerbaren Betätigungshandhabe (82),
wobei
- die Betätigungshandhabe (82) mit dem Flüssigkeitsspeicher (80) derart mechanisch gekoppelt ist, dass eine Verlagerung der Betätigungshandhabe (82) eine Volumenreduktion des Flüssigkeitsspeichers (80) bewirkt,
**dadurch gekennzeichnet, dass**
- dem Auslasskanal (58, 56, 70, 74) eine Ventileinrichtung (54, 72) zugeordnet ist, die in einem Öffnungszustand die Verbindung des Flüssigkeitsspeichers (80) mit der Austragöffnung (66) freigibt und in einem Schließzustand die Verbindung des Flüssigkeitsspeichers (88) mit der Austragöffnung (66) trennt, und
- die Ventileinrichtung (54, 72) eine von außen zugängliche Ventilhandhabe (68) aufweist, mittels derer die Ventileinrichtung (54, 72) von außen aus dem Öffnungszustand in den Schließzustand überführbar ist.

2. Austragvorrichtung (10) nach Anspruch 1,
**dadurch gekennzeichnet, dass**
- der Flüssigkeitsspeicher (80) einteilig mit der Betätigungshandhabe (82) ausgebildet ist und
- ein zum Außengehäuse (20) ortsfester Kolbenabschnitt (46) vorgesehen ist, der bei einer über die Betätigungshandhabe (82) bewirkten Verlagerung des Flüssigkeitsspeichers (80) in den Flüssigkeitsspeicher (80) einrückt.

3. Austragvorrichtung nach Anspruch 1 oder 2,
**dadurch gekennzeichnet, dass**
die Ventileinrichtung (54, 72) zwei gegeneinander verlagerbare Ventilabschnitte (54, 72) aufweist, die im Schließzustand der Ventileinrichtung (54, 72) aneinander anliegend den Auslasskanal (58, 56, 70, 74) sperren, wobei die Ventilhandhabe (68) vorzugsweise einteilig, insbesondere einstückig, mit einem der Ventilabschnitte (72) ausgebildet ist.

4. Austragvorrichtung nach Anspruch 3,
**dadurch gekennzeichnet, dass**
- die Ventilabschnitte (54, 72) in einer Verlagerungsrichtung (6a, 6b) gegeneinander verlagerbar sind,
- ein erster Ventilabschnitt (54) als in Verlagerungsrichtung (6a, 6b) erstreckter zylindrischer oder konischer Ventilstift (54) und ein zweiter Ventilabschnitt (72) als eine zumindest im Schließzustand den Ventilstift (54) anliegend umgebende und in Verlagerungsrichtung (6a, 6b) erstreckte zylindrische oder konische Ventilhülse (72) ausgebildet ist und
- die Ventilhülse (72) in Radialrichtung erstreckte Verwirbelungsdurchbrechungen (74) aufweist.

5. Austragvorrichtung nach einem der vorstehenden Ansprüche,
**dadurch gekennzeichnet, dass**
die Austragöffnung (66) in einem gegenüber dem Außengehäuse (20) verlagerbaren Ventilabschnitt (60) vorgesehen ist.

6. Austragvorrichtung nach einem der vorstehenden Ansprüche,
**dadurch gekennzeichnet, dass**
die Ventileinrichtung (54, 72) als bistabile Ventileinrichtung (54, 72) ausgebildet ist, die ohne extern Kraft- oder Druckbeaufschlagung den Schließzustand und den Öffnungszustand aufrechterhält.

7. Austragvorrichtung nach einem der vorstehenden Ansprüche,
**dadurch gekennzeichnet, dass**
die Ventileinrichtung (54, 72) dafür ausgebildet ist, durch Kraftbeaufschlagung der Betätigungshandhabe (82) aus dem Schließzustand in den Öffnungszustand überführbar zu sein.

8. Austragvorrichtung nach einem der vorstehenden Ansprüche,
**dadurch gekennzeichnet, dass**
die Ventilhandhabe (68) derart ausgebildet und/oder angeordnet ist, dass von außen und mittels der Ventilhandhabe (68) keine Überführung der Ventileinrichtung (54, 72) aus dem Schließzustand in den Öffnungszustand möglich ist.

9. Austragvorrichtung nach Anspruch 8,
**dadurch gekennzeichnet, dass**
die Ventilhandhabe (68) in einer Durchbrechung (26) des Außengehäuses (20) angeordnet ist und im Schließzustand der Ventileinrichtung (54, 72) gegenüber dem einer Außenfläche des Außengehäuses (20) zumindest partiell zurückgesetzt ist.

10. Montageverfahren für eine Austragvorrichtung nach Anspruch 1,
**dadurch gekennzeichnet, dass**
a. die Ventileinrichtung (54, 72) in ihren Öffnungszustand versetzt wird,
b. der zumindest teilweise mit Flüssigkeit gefüllte Flüssigkeitsspeicher (80) an den Auslasskanal (58, 56, 70, 74) angekoppelt wird, so dass der Flüssigkeitsspeicher (80) mit einer Außenumgebung nur noch über die Austragöffnung (66) verbunden ist,
c. das Volumen des Flüssigkeitsspeichers (80) anschließend verringert wird und
d. die Ventileinrichtung (54, 72) anschließend in ihren Schließzustand versetzt wird.

## Geänderte Patentansprüche

### Geänderte Patentansprüche gemäss Regel 137(2) EPÜ.

**1.** Austragvorrichtung (10) mit
- einem Außengehäuse (20),
- einer Austragöffnung (66),
- einem Flüssigkeitsspeicher (80),
- einem Auslasskanal (58, 56, 70, 74), durch den der Flüssigkeitsspeicher (80) mit der Austragöffnung (66) verbunden ist, und
- einer gegenüber dem Außengehäuse (20) verlagerbaren Betätigungshandhabe (82),
wobei
- die Betätigungshandhabe (82) mit dem Flüssigkeitsspeicher (80) derart mechanisch gekoppelt ist, dass eine Verlagerung der Betätigungshandhabe (82) eine Volumenreduktion des Flüssigkeitsspeichers (80) bewirkt,
- dem Auslasskanal (58, 56, 70, 74) eine Ventileinrichtung (54, 72) zugeordnet ist, die in einem Öffnungszustand die Verbindung des Flüssigkeitsspeichers (80) mit der Austragöffnung (66) freigibt und in einem Schließzustand die Verbindung des Flüssigkeitsspeichers (88) mit der Austragöffnung (66) trennt, und
- die Ventileinrichtung (54, 72) eine von außen zugängliche Ventilhandhabe (68) aufweist, mittels derer die Ventileinrichtung (54, 72) von außen aus dem Öffnungszustand in den Schließzustand überführbar ist,
**dadurch gekennzeichnet, dass**
die Ventileinrichtung (54, 72) als bistabile Ventileinrichtung (54, 72) ausgebildet ist, die ohne externe Kraft- oder Druckbeaufschlagung den Schließzustand und den Öffnungszustand aufrechterhält und durch Kraftbeaufschlagung der Betätigungshandhabe (82) aus dem Schließzustand in den Öffnungszustand überführbar ist.

**2.** Austragvorrichtung (10) nach Anspruch 1,
**dadurch gekennzeichnet, dass**
- der Flüssigkeitsspeicher (80) einteilig mit der Betätigungshandhabe (82) ausgebildet ist und
- ein zum Außengehäuse (20) ortsfester Kolbenabschnitt (46) vorgesehen ist, der bei einer über die Betätigungshandhabe (82) bewirkten Verlagerung des Flüssigkeitsspeichers (80) in den Flüssigkeitsspeicher (80) einrückt.

**3.** Austragvorrichtung nach Anspruch 1 oder 2,
**dadurch gekennzeichnet, dass**
die Ventileinrichtung (54, 72) zwei gegeneinander verlagerbare Ventilabschnitte (54, 72) aufweist, die im Schließzustand der Ventileinrichtung (54, 72) aneinander anliegend den Auslasskanal (58, 56, 70, 74) sperren, wobei die Ventilhandhabe (68) vorzugsweise einteilig, insbesondere einstückig, mit einem der Ventilabschnitte (72) ausgebildet ist.

**4.** Austragvorrichtung nach Anspruch 3,
**dadurch gekennzeichnet, dass**
- die Ventilabschnitte (54, 72) in einer Verlagerungsrichtung (6a, 6b) gegeneinander verlagerbar sind,
- ein erster Ventilabschnitt (54) als in Verlagerungsrichtung (6a, 6b) erstreckter zylindrischer oder konischer Ventilstift (54) und ein zweiter Ventilabschnitt (72) als eine zumindest im Schließzustand den Ventilstift (54) anliegend umgebende und in Verlagerungsrichtung (6a, 6b) erstreckte zylindrische oder konische Ventilhülse (72) ausgebildet ist und
- die Ventilhülse (72) in Radialrichtung erstreckte Verwirbelungsdurchbrechungen (74) aufweist.

**5.** Austragvorrichtung nach einem der vorstehenden Ansprüche,
**dadurch gekennzeichnet, dass**
die Austragöffnung (66) in einem gegenüber dem Außengehäuse (20) verlagerbaren Ventilabschnitt (60) vorgesehen ist.

**6.** Austragvorrichtung nach einem der vorstehenden Ansprüche,
**dadurch gekennzeichnet, dass**
die Ventilhandhabe (68) derart ausgebildet und/oder angeordnet ist, dass von außen und mittels der Ventilhandhabe (68) keine Überführung der Ventileinrichtung (54, 72) aus dem Schließzustand in den Öffnungszustand möglich ist.

**7.** Austragvorrichtung nach Anspruch 6,
**dadurch gekennzeichnet, dass**
die Ventilhandhabe (68) in einer Durchbrechung (26) des Außengehäuses (20) angeordnet ist und im Schließzustand der Ventileinrichtung (54, 72) gegenüber dem einer Außenfläche des Außengehäuses (20) zumindest partiell zurückgesetzt ist.

**8.** Montageverfahren für eine Austragvorrichtung nach Anspruch 1,
**dadurch gekennzeichnet, dass**
a. die Ventileinrichtung (54, 72) in ihren Öffnungszustand versetzt wird,
b. der zumindest teilweise mit Flüssigkeit gefüllte Flüssigkeitsspeicher (80) an den Auslasskanal (58, 56, 70, 74) angekoppelt wird, so dass der Flüssigkeitsspeicher (80) mit einer Außenumgebung nur noch über die Austragöffnung (66) verbunden ist,
c. das Volumen des Flüssigkeitsspeichers (80) anschließend verringert wird und
d. die Ventileinrichtung (54, 72) anschließend in ihren Schließzustand versetzt wird.
